# EUROPEAN PATENT APPLICATION

(11) **EP 4 317 984 A1**
(43) Date of publication of application: **07.02.2024**
(21) Application number: 22775055.1
(22) Date of filing: 07.03.2022
(51) Int. Cl.: G01N 35/00, G01N 33/543

(54) **IMMUNOCHROMATOGRAPHIC TEST DEVICE**

(30) Priority: 24.03.2021 JP 2021050776
(71) Applicant: FUJIFILM Corporation, Tokyo 106-8620 (JP)
(72) Inventor: TANAKA, Yasutake, Ashigarakami-gun, Kanagawa 258-8538 (JP)
(74) Representative: Hughes, Andrea Michelle
(86) International application number: PCT/JP2022/009821
(87) International publication number: WO 2022/202261

(57) **Abstract**

The immunochromatographic assay apparatus includes a loading part in which a cartridge including a carrier and a second reagent holding part is attachably and detachably loaded, in which the carrier has at least three regions of a spotting region on which a sample is spotted, an assay region in which a color development state changes depending on whether the sample is positive or negative, and a color development region in which a color development state changes by reaction with a first reagent, and in which the second reagent holding part holds a second reagent to be developed in the assay region after the first reagent has been developed in the color development region, a detection unit that detects a color development state in the color development region, a second reagent supply mechanism for starting a supply of the second reagent from the second reagent holding part to the carrier, and a processor configured to operate the second reagent supply mechanism based on a change in the color development state of the color development region.

## Description

### BACKGROUND OF THE INVENTION

### 1. Field of the Invention

The present disclosure relates to an immunochromatographic assay apparatus.

### 2. Description of the Related Art

Among immunoassay methods, an immunochromatographic method is generally widely utilized, because operation is easy and assay can be performed within a short period of time.

WO2016/114122A and WO2017/104143A disclose an immunochromatographic kit using an immunochromatographic method. The immunochromatographic kit includes an immunochromatographic carrier to which a sample is supplied. The immunochromatographic carrier provides with an assay region on which an antibody that specifically binds to an antigen, which is a test substance, is immobilized. In a case where a labeled antibody that specifically binds to an antigen is developed on an immunochromatographic carrier together with a sample containing the antigen, the antigen binds to the antibody immobilized on the assay region and the labeling substance is captured via the antigen. In a case where the assay region develops a color by the labeling substance captured in the assay region, it is determined that the sample is positive. In a case where the amount of the labeling substance captured in the assay region is very small, the color development is weak and it may be determined that the sample is false negative. Therefore, WO2016/114122A and WO2017/104143A disclose an amplification technique for amplifying a labeling signal emitted by a labeling substance. The disclosed amplification technique is a silver amplification technique in which gold colloidal particles are used as a labeling substance and silver ions and a silver ion reducing agent are used as reagents for amplification. In the silver amplification, an amplification reaction is caused in which using the gold colloidal particles as a catalyst, silver particles having a relatively large particle diameter are generated. By this amplification reaction, the labeling signal emitted by the gold colloidal particles is amplified.

The immunochromatographic kits according to WO2016/114122A and WO2017/104143A include a first amplifying liquid pod holding a first amplifying liquid (corresponding to a first reagent) containing a silver ion reducing agent, and a second amplifying liquid that holds a second amplifying liquid (corresponding to a second reagent) containing silver ions. The immunochromatographic kit includes an operation structure such as an operation button for applying a pressing force to the first amplifying liquid pod and an operation button for applying a pressing force to the second amplifying liquid pod. By applying a pressing force through each operation structure, it is possible to supply the first amplifying liquid and the second amplifying liquid to the immunochromatographic carrier to cause an amplification reaction.

On the other hand, JP2012-103150A discloses an analysis apparatus that includes a loading part in which a cartridge corresponding to an immunochromatographic kit is loaded and optically analyzes a reaction state between a sample and a reagent in an assay region. The analysis apparatus includes a sensor that optically detects a reaction state and a display unit that displays the detection result. By using the analysis apparatus, it is possible for the user to perform determination mechanically whether the sample is a positive or negative only by performing the operation of loading the cartridge in which the sample is spread. The cartridge described in JP2012-103150A includes an amplifying liquid pod, and the analysis apparatus includes, in addition to the sensor and the display unit, an internal mechanism such as a pressing mechanism for pressing the amplifying liquid pod. Therefore, in the analysis apparatus, the amplifying liquid pod is pressed by the internal mechanism, and thus the amplifying liquid is supplied from the amplifying liquid pod to the immunochromatographic carrier.

### SUMMARY OF THE INVENTION

As the immunochromatographic kits (corresponding to a cartridges) described in WO2016/114122A and WO2017/104143A, an assay apparatus using a cartridge including a first amplifying liquid pod and a second amplifying liquid pod is also known. In the conventional assay apparatus, in order to supply the second reagent after the first reagent is developed, after the supply of the first reagent is started, the supply timing of the second reagent is temporally managed by using a timer. In a case where such the supply timing of the second reagent is temporally managed, the time from the start of supply of the first reagent to the completion of the determination is constant regardless of the individual difference for each cartridge.

However, there may be individual differences in the development time of the first reagent for each cartridge, for example, in some cases, the development time of the first reagent is long and in some cases, the development time of the first reagent is short. In a case of temporally managing the supply timing of the second reagent in consideration of such individual differences, it is necessary to set a waiting time until the supply of the second reagent in accordance with the cartridge in which the development time of the first reagent is the longest. In that case, even in a cartridge in which the development time of the first reagent is relatively short, the assay apparatus is occupied for a time same as the cartridge in which the development time of the first reagent is the longest. In a case where a plurality of cartridges are assayed, in order to improve the throughput, it is desired to shorten the occupancy time in which the assay apparatus is occupied by the cartridge, as much as possible.

The analysis apparatus of JP2012-103150A also manages the time from spotting of the sample on the cartridge to the determination. JP2012-103150A also has the same problems as those of WO2016/114122A and WO2017/104143A because the determination is performed by time management without considering the individual difference of the cartridge.

The present disclosure has been made in view of the above circumstances, and an object of the present disclosure is to provide an immunochromatographic assay apparatus capable of shortening the occupancy time of the immunochromatographic assay apparatus by the cartridge according to an individual difference as compared with the prior art in which the supply timing of the second reagent is temporally managed by using a timer.

The immunochromatographic assay apparatus of the present disclosure includes a loading part in which a cartridge including a carrier and a second reagent holding part is attachably and detachably loaded, in which the carrier has at least three regions of a spotting region on which a sample is spotted, an assay region in which a color development state changes depending on whether the sample is positive or negative, and a color development region in which a color development state changes by reaction with a first reagent, and in which the second reagent holding part holds a second reagent to be developed in the assay region after the first reagent has been developed in the color development region,
a detection unit that detects a color development state in the color development region,
a second reagent supply mechanism for starting a supply of the second reagent from the second reagent holding part to the carrier, and
a processor configured to discriminate, based on a change in the color development state of the color development region, presence or absence of the change in the color development state of the color development region and operate the second reagent supply mechanism.

In the immunochromatographic assay apparatus of the present disclosure, at least the second reagent among the first reagent and the second reagent is preferably an amplifying liquid that amplifies color development in the assay region.

In the immunochromatographic assay apparatus of the present disclosure, the first reagent and the second reagent may be amplifying liquids that amplify color development in the assay region by reacting with each other.

In the immunochromatographic assay apparatus of the present disclosure, in a case where the change in the color development state of the color development region is discriminated to be absent, after the discrimination, the processor is preferably configured to discriminate the presence or absence of the change in the color development state of the color development region after a lapse of a preset first setting time.

In the immunochromatographic assay apparatus of the present disclosure, in a case where the color development state of the color development region is not changed even after a lapse of a preset second setting time from a preset time point after the cartridge is loaded or even after repeating a preset number of times of discrimination, the processor is preferably configured to notify an error.

In the immunochromatographic assay apparatus of the present disclosure, preferably, in a case where a direction toward the assay region with respect to the spotting region is defined as a downstream side of the carrier, the carrier of the cartridge has a control region that is provided on a downstream side of the assay region and that shows a development of the sample supplied from the spotting region to the carrier in the assay region by a change in color development state, in a case where the detection unit is defined as a first detection unit, the color development state detected by the first detection unit is defined as a first color development state, the color development state of the control region is defined as a second color development state, and the color development state of the assay region is defined as a third color development state, the immunochromatographic assay apparatus includes a second detection unit that detects the second color development state of the control region, and a third detection unit that detects the third color development state of the assay region, and in a case where a change in the first color development state of the color development region is present and a change in the second color development state of the control region is discriminated to be present, the processor is configured to discriminate presence or absence of a change in the third color development state of the assay region.

In the immunochromatographic assay apparatus of the present disclosure, the processor is preferably configured to operate the second reagent supply mechanism only in a case where the change in the first color development state of the color development region is discriminated to be present and the change in the second color development state of the control region is absent.

In the immunochromatographic assay apparatus of the present disclosure, after operating the second reagent supply mechanism, in a case where the change in the second color development state of the control region is absent even after a lapse of a preset third setting time, the processor is preferably configured to notify an error.

In the immunochromatographic assay apparatus of the present disclosure, the cartridge preferably includes a first reagent holding part that holds the first reagent.

In the immunochromatographic assay apparatus of the present disclosure, the cartridge in a state where a supply of the first reagent from the first reagent holding part to the carrier is started is preferably capable of being loaded.

The immunochromatographic assay apparatus of the present disclosure preferably includes a first reagent supply mechanism for causing the cartridge in a state of being loaded in the loading part to start a supply of the first reagent from the first reagent holding part to the carrier.

In the immunochromatographic assay apparatus of the present disclosure, preferably, in the cartridge, in a case where a direction toward the assay region with respect to the spotting region is defined as a downstream side of the carrier, the color development region is disposed on a downstream side of the assay region and the first reagent holding part is provided on an upstream side of the spotting region.

In the immunochromatographic assay apparatus of the present disclosure, preferably, in a case where the color development region disposed on a downstream side of the assay region is defined as a downstream-side color development region, the carrier of the cartridge has an upstream-side color development region which is on an upstream side of the assay region and is disposed between the first reagent holding part and the assay region, and in which a color development state changes by a reaction with the first reagent, in a case where the detection unit is defined as a downstream-side detection unit that detects a color development state of the downstream-side color development region, the immunochromatographic assay apparatus includes an upstream-side detection unit that detects a color development state of the upstream-side color development region, and in a case where a change in the color development state of the upstream-side color development region is discriminated to be absent, the processor is configured to operate the first reagent supply mechanism.

According to the immunochromatographic assay apparatus of the present disclosure, the occupancy time of the immunochromatographic assay apparatus by the cartridge can be shortened according to an individual difference of the cartridge as compared with the prior art in which the supply timing of the second reagent is temporally managed by using a timer.

### BRIEF DESCRIPTION OF THE DRAWINGS

Fig. 1 is a perspective view showing appearance of an immunochromatographic assay apparatus.
Fig. 2 is a perspective view of a cartridge.
Fig. 3 is an exploded perspective view of the cartridge.
Fig. 4 is a diagram showing a positional relationship between an assay strip, a multifunctional member, a first reagent holding part, and a second reagent holding part, in the cartridge.
Fig. 5 is an explanatory diagram of an immunochromatographic method.
Fig. 6 is a partially broken side view of an assay apparatus in a state where the cartridge is loaded.
Fig. 7 is a partially broken side view of the assay apparatus in a state where the cartridge is loaded and a second reagent supply mechanism is operated.
Fig. 8 is a diagram showing a first assay flow.
Fig. 9 is a diagram showing a first assay flow in an assay apparatus.
Fig. 10 is a diagram showing a second assay flow in an assay apparatus.
Fig. 11 is a side view of an assay strip 1A of a design change example.
Fig. 12 is a diagram showing a third assay flow in an assay apparatus of the design change example.

### DESCRIPTION OF THE PREFERRED EMBODIMENTS

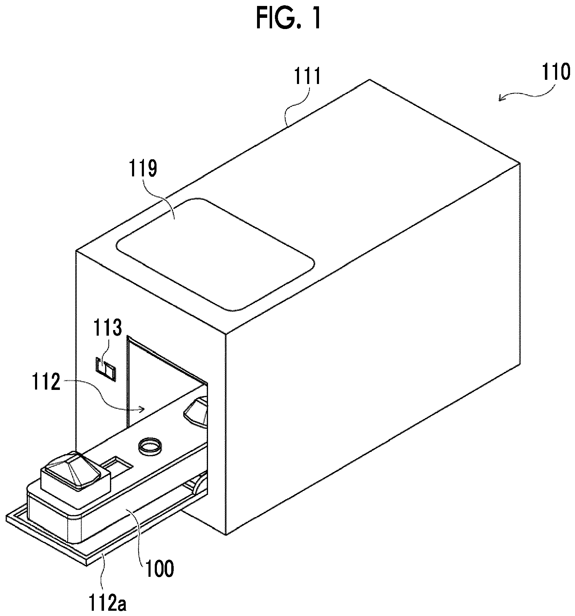

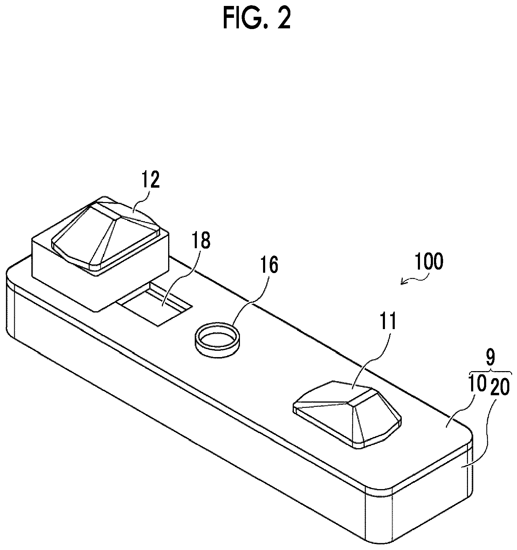

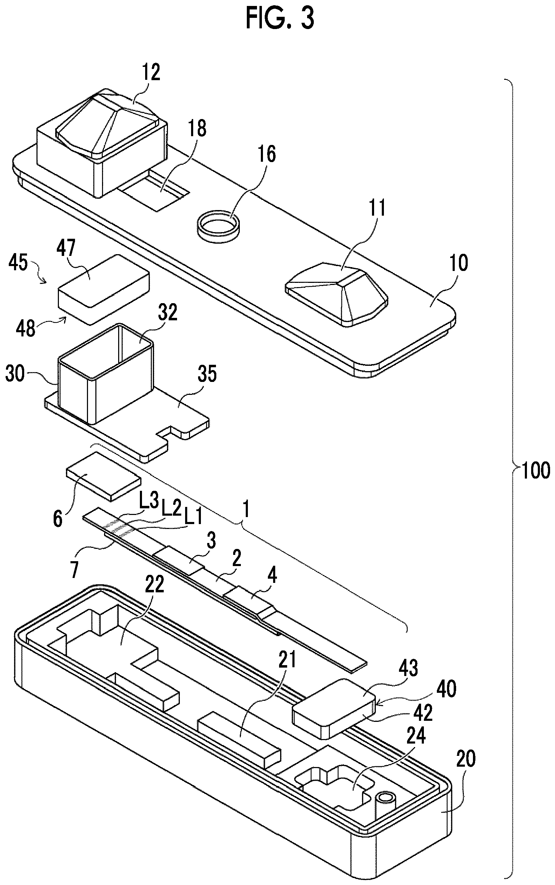

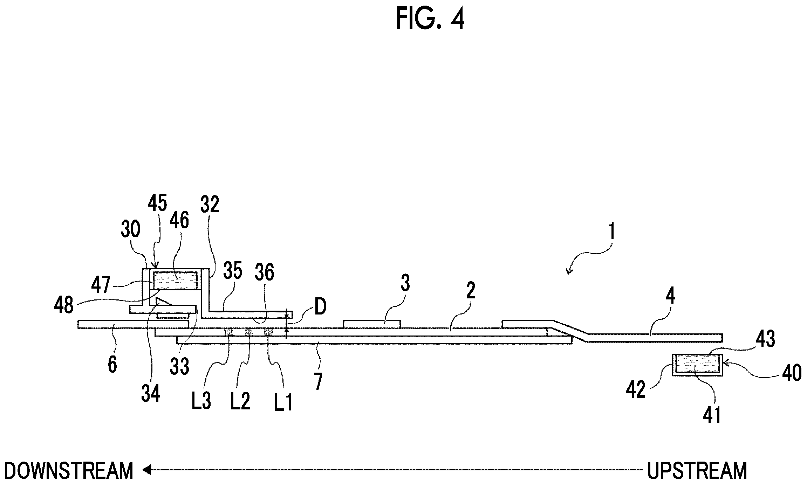

Embodiments of the immunochromatographic assay apparatus of the present disclosure will be described with reference to the drawings. Fig. 1 is a perspective view illustrating the appearance of the immunochromatographic assay apparatus 110 (hereinafter, simply referred to as an assay apparatus 110) according to one embodiment. Fig. 2 is an external view of a cartridge 100 mounted on the assay apparatus 110 and Fig. 3 is an exploded perspective view of the cartridge 100. Fig. 4 is a diagram illustrating the positional relationship of the main accommodated components in the cartridge 100.

The cartridge 100 is a single-use type that is used one by one for each sample of assay target. As shown in Fig. 3, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state in which the sample is spotted is loaded. Then, the assay apparatus 110 detects a color development state of the assay region L1 of the loaded cartridge 100, and presents the result of whether the sample is positive or negative. In a case of where a plurality of samples are assayed, the cartridge 100 for each sample is loaded one by one into the assay apparatus 110.

Hereinafter, the cartridge 100 will be described on the premise that the cartridge 100 is loaded into the assay apparatus 110. However, the cartridge 100 of the present example has a configuration that a user can confirm visually whether the sample is positive or negative without using the assay apparatus 110. Such a cartridge 100 is also referred to as an immunochromatographic assay tool, an immunochromatographic assay kit, or the like.

As shown in Fig. 1, the assay apparatus 110 includes a case body 111, and the case body 111 includes a cartridge loading part 112 in which the cartridge 100 is attachably and detachably loaded. As an example, an opening for inserting the cartridge 100 into the case body 111 and an opening/closing lid 112a for opening and closing the opening are provided on the front surface of the case body 111. The opening/closing lid 112a is opened when the cartridge 100 is loaded, the cartridge 100 is inserted into the case body 111, and the opening/closing lid 112a is closed after the cartridge 100 has been loaded into the cartridge loading part 112. The assay is performed in a state where the opening/closing lid 112a is closed.

In addition, a power switch 113 is provided on the front surface of the case body 111, and a monitor 119 is provided on the upper surface of the case body 111. A determination result, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

As shown in Fig. 2 and Fig. 3, as an example, the cartridge 100 includes a housing 9 constituted of a case member 20 and a cover member 10. The housing 9 is formed of, for example, a resin material. An opening is formed in an upper part of the case member 20, and in addition to the assay strip 1, a first reagent holding part 40, a second reagent holding part 45, and the like are accommodated therein. The cover member 10 covers the opening of the case member 20 by being attached to the opening part of the case member 20. The housing 9 has an elongated shape as a whole in accordance with the elongated shape of the assay strip 1.

In the present example, a dropping port 16, an observation window 18, a first pressed part 11, and a second pressed part 12 are provided on an upper part of the housing 9 constituted of the cover member 10. Each of these parts is integrally molded with the cover member 10 as an example. The dropping port 16 is an opening for adding dropwise a sample into the inside of the housing 9. A boss is vertically provided on the edge of the dropping port 16 toward the upper part. The observation window 18 is a window for observing the assay region L1 from the outside, and is formed of a transparent member as an example. In the present example, the size of the observation window 18 is a size such that, in addition to the assay region L1, the control region L2 and the color development region L3, which will be described later, can also be observed.

The first pressed part 11 is an operating part operated to supply the first reagent 41 (see Fig. 4) in the first reagent holding part 40 to the carrier 2. The second pressed part 12 is an operating part operated to supply the second reagent 46 (see Fig. 4) in the second reagent holding part 45 to the carrier 2. As will be described later, the first reagent 41 and the second reagent 46 are amplifying liquids for amplifying the color development in the assay region L1 in a case where the sample is positive.

In a case where a pressing force is applied from the outside as an external force to the first pressed part 11, the first pressed part 11 is deformed. As an example, the first pressed part 11 has a quadrangular pyramid shape, and in a case where a pressing force is applied from above to a region including the apex of the quadrangular pyramid, the first pressed part 11 is deformed such that the apex of the quadrangular pyramid sinks into the inside of the housing 9. In a case of where the first pressed part 11 is deformed in this manner, a pressing force is applied to the first reagent holding part 40 inside the housing 9. In the first reagent holding part 40, deformation or the like due to a pressing force applied through the first pressed part 11 occurs. Due to this deformation or the like, the first reagent 41 held by the first reagent holding part 40 is supplied to the assay strip 1.

In addition, it is preferable that the first pressed part 11 is deformed by pressing and then the deformed state is maintained. The reason is as follows. As will be described later, in the assay apparatus 110 of the present example, the cartridge 100 in a state in which the first pressed part 11 is pressed in advance by the user can be loaded. It is because, in a case where the first pressed part 11 is pressed by the user before being loaded into the assay apparatus 110, the first pressed part 11 in which the deformation is maintained even after the user releases the hand, is easier to continue the supply of the first reagent 41.

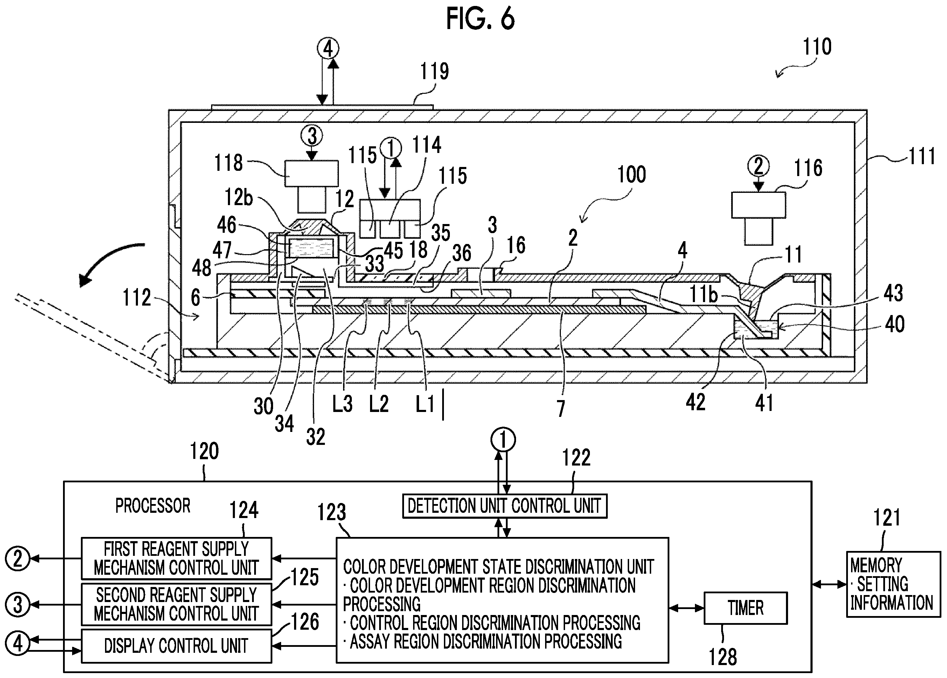

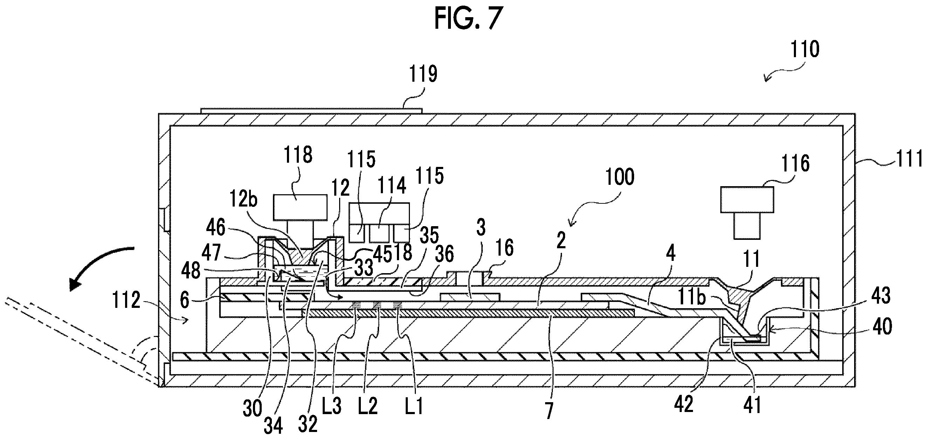

Similarly, in a case where a pressing force is applied from the outside as an external force to the second pressed part 12, the second pressed part 12 is deformed. Similarly to the first pressed part 11, the second pressed part 12 of the present example also has a quadrangular pyramid shape, and in a case where a pressing force is applied from above to a region including the apex of the quadrangular pyramid, the second pressed part 12 is deformed such that the apex of the quadrangular pyramid sinks into the inside of the housing 9. In a case of where the second pressed part 12 is deformed in this manner, a pressing force is applied to the second reagent holding part 45 inside the housing 9. In the second reagent holding part 45, deformation or the like due to a pressing force applied through the second pressed part 12 occurs. Due to this deformation or the like, the second reagent 46 held by the second reagent holding part 45 is supplied to the assay strip 1. In the second pressed part 12 of the present example, an abutting part 12b that abuts on the second reagent holding part 45 is provided (see Fig. 6 and Fig. 7).

In addition, in a case where the cartridge 100 is assayed by the assay apparatus 110, unlike the first pressed part 11, the second pressed part 12 is pressed by the internal mechanism of the assay apparatus 110 in any of the assay flows that can be selected in the assay apparatus 110. Therefore, the second pressed part 12 may be depressible by an internal mechanism. Of course, a case where the cartridge 100 is used without using the assay apparatus 110 is preferably an aspect that the second pressed part 12 is also depressible by the user.

As shown in Fig. 3 and Fig. 4, the case member 20 accommodates the assay strip 1 including the carrier 2 along the longitudinal direction. In the case member 20, the first reagent holding part 40 is disposed on one end part side (upstream side shown in Fig. 4) in the longitudinal direction. In the case member 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

The first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward. The pressing force applied from the first pressed part 11 is transmitted to the sheet member 43 of the first reagent holding part 40 via the end part of the assay strip 1 to break the sheet member 43 (see Fig. 6 and Fig. 7). The sheet member 43 is broken, and thus the first reagent 41 is supplied to the assay strip 1. In the first pressed part 11 of the present example, a protruding part 11b that abuts on the sheet member 43 (see Fig. 6 and Fig. 7). The protruding part 11b has, for example, an elongated shape extending in the longitudinal direction in the width direction of the assay strip 1 and a pointed shape toward the sheet member 43, such that the sheet member 43 is easily broken.

In addition, the cartridge 100 includes a multifunctional member 30 having a function of accommodating the second reagent holding part 45. The multifunctional member 30 is disposed on the other end part side (downstream side shown in Fig. 4) of the case member 20 and above the assay strip 1. The multifunctional member 30 is a member in which the second accommodating part 32 and the flow channel forming part 35 are integrally formed. The second accommodating part 32 is a part accommodating the second reagent holding part 45. The second accommodating part 32 has a box shape having an opened upper surface. As shown in Fig. 4, on the bottom of the second accommodating part 32, a protrusion 34 for breaking a sheet member 48, which will be described later, of the second reagent holding part 45, and an opening 33 that allows to flow the second reagent 46 flowed out from the second reagent holding part 45, toward the assay strip 1.

Furthermore, the flow channel forming part 35 is provided to be connected to the upstream side from the second accommodating part 32. The flow channel forming part 35 has a flat plate shape, is disposed at a position facing the assay region L1 or the like in the longitudinal direction of the assay strip 1, and is disposed with an interval from the assay strip 1. Then, between the flow channel forming part 35 and the assay strip 1, a flow channel for flowing the second reagent 46 flowed out from the second accommodating part 32 toward the assay region L1 or the like is formed. In this way, the flow channel forming part 35 is disposed between the observation window 18 and the assay region L1 or the like of the assay strip 1. Therefore, the flow channel forming part 35 is formed of a transparent member and thus the assay region L1 and the like can be observed through the observation window 18.

The second reagent holding part 45 holds the second reagent 46. The second reagent holding part 45 is constituted of, for example, a container 47 formed of a resin material and having an opening on one surface, and a sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion 34 in the second accommodating part 32.

The pressing force applied from the second pressed part 12 to the second reagent holding part 45 acts in a direction of pushing down the second reagent holding part 45 downwardly, whereby the sheet member 48 is pressed against the protrusion 34. The sheet member 48 is pressed against the protrusion 34, whereby the sheet member 48 is broken (see Fig. 6 and Fig. 7). The sheet member 48 is broken, and thus the second reagent 46 is supplied to the assay strip 1 through the flow channel formed by the opening 33 at the bottom of the second accommodating part 32 and the flow channel forming part 35.

As shown in Fig. 4, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the opening 33 at the bottom of the second accommodating part 32 toward the carrier 2, and the second reagent 46 that has flowed out flows through the flow channel formed by the gap D and reaches at least above the assay region L1. The second reagent 46 that has reached the assay region L1 infiltrates the assay region L1 from the flow channel.

An absorption pad 6, which will be described later, is disposed at an end part on the downstream side of the assay strip 1. In the case member 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case member 20, a support part 21 that supports a central part of the assay strip 1 is formed.

The assay strip 1 includes a carrier 2, a liquid feeding pad 4, and an absorption pad 6. Then, the carrier 2 is fixedly supported on a back pressure-sensitive adhesive sheet 7.

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

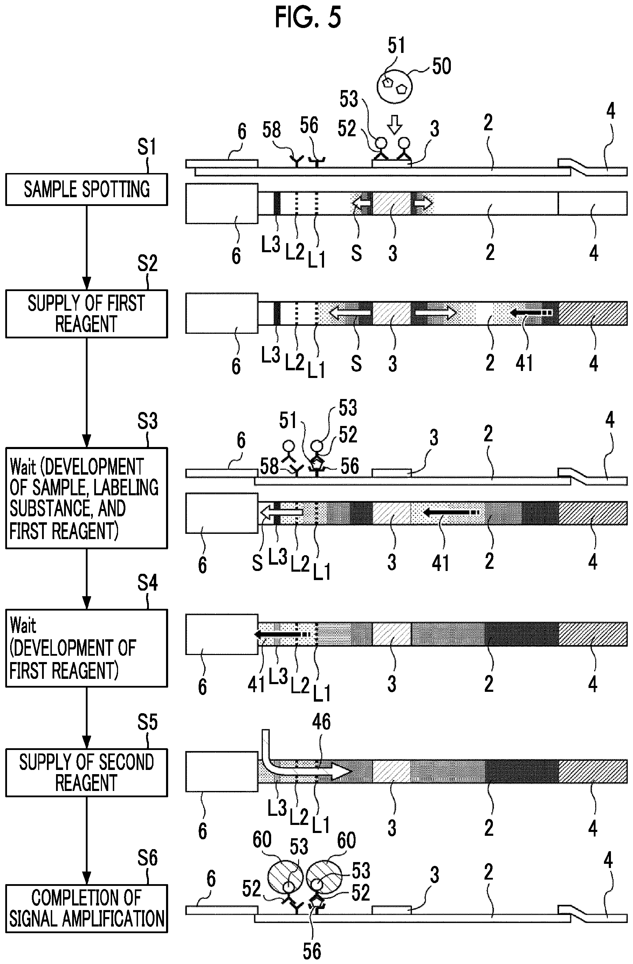

It shows a state in which the assay region L1, the control region L2, and the color development region L3 are expressed as lines, but these are not always expressed. Details will be described later, but before developing the sample 50 (see Fig. 5), the first reagent 41 (see Fig. 4), and the second reagent 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. As a result, the assay region L1 becomes visible. Since the color development of the assay region L1 is amplified by silver amplification, which will be described later, the assay region L1 develops a black color.

The control region L2 is also expressed as a line by increasing the color optical density in a case where the sample 50 is developed. As a result, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is fixed in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

The liquid feeding pad 4 is disposed in contact with one end of the carrier 2 and the first reagent 41 is fed to the carrier 2 from the upstream side of the spotting region (constituted of the label holding pad 3). In the liquid feeding pad 4, in a case where the first pressed part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary phenomenon.

The absorption pad 6 is disposed in contact with the other end of the carrier 2 and absorbs the sample 50, the first reagent 41, and the second reagent 46, which are developed on the carrier 2. The absorption pad 6 is also formed of a porous material.

In the present embodiment, the first reagent 41 and the second reagent 46 are amplifying liquids that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplifying liquids used for silver amplification, and the reaction between the first reagent 41 and the second reagent 46 using the labeling substance 53 as a catalyst is an amplification reaction. By the amplification reaction, silver particles 60 (see Fig. 5) having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 (see Fig. 5) are generated, and the generated silver particles 60 deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. The sample 50 is developed on the downstream side from the label holding pad 3 in the carrier 2 by the capillary phenomenon in the carrier 2. A part of the sample 50 is also developed on the upstream side. The arrow S indicates a state in which the sample 50 is developed.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 5 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

Fig. 6 and Fig. 7 are partially broken side views of the assay apparatus 110 in a state where the cartridge 100 is loaded. Hereinafter, a configuration and a function of the assay apparatus 110 will be described with reference to Fig. 6 and Fig. 7.

In the assay apparatus 110 of the present example, as shown below, the two assay flows of a first assay flow and a second assay flow can be selected. In both of the first assay flow and the second assay flow, it is necessary that the sample 50 is spotted on the carrier 2 of the cartridge 100 before loading. However, in a case where the first assay flow is selected, it is necessary that the supply of the first reagent 41 to the carrier 2 in the cartridge 100 is started by the operation of the user before loading, but in a case where the second assay flow is selected, the supply is started by the internal mechanism of the assay apparatus 110 after the loading.

That is, the first assay flow is a flow in which an assay is performed on the cartridge 100 in a state where the spotting of the sample 50 and the supply of the first reagent 41 are started before loading. In the case of the first assay flow, after the loading, only the supply of the second reagent 46 to the carrier 2, among the first reagent 41 and the second reagent 46, is performed by the assay apparatus 110.

The second assay flow is a flow in which an assay is performed on the cartridge 100 in which only the spotting of the sample 50 is performed before loading. In the case of the second assay flow, after the loading, the supply of both of the first reagent 41 and the second reagent 46 to the carrier 2 is performed by the assay apparatus 110.

Hereinafter, after a configuration of the assay apparatus 110 is described, the first assay flow will be described first, and then the second assay flow will be described.

### (Configuration of assay apparatus 110)

As shown in Fig. 6 and Fig. 7, the assay apparatus 110 includes a first reagent supply mechanism 116 and a second reagent supply mechanism 118 as internal mechanisms. The first reagent supply mechanism 116 is a mechanism for starting the supply of the first reagent 41 from the first reagent holding part 40 to the carrier 2. As the first reagent supply mechanism 116, for example, an actuator such as a solenoid provided with an electromagnet and a plunger movable with respect to the electromagnet is used. For example, as the plunger moves, the plunger abuts on the first pressed part 11 and presses the first pressed part 11. The first reagent supply mechanism 116 is disposed at a position facing the first pressed part 11 of the loaded cartridge 100.

The first reagent supply mechanism 116 is a pressing mechanism that applies a pressing force to the first pressed part 11 from the outside by pressing the first pressed part 11 of the cartridge 100. In a case where a pressing force is applied to the first pressed part 11 by the first reagent supply mechanism 116, the first reagent 41 is supplied from the first reagent holding part 40 to the carrier 2 by the above-described action. In the first assay flow, the first reagent supply mechanism 116 is not used and only in the second assay flow, the first reagent supply mechanism 116 is used.

The second reagent supply mechanism 118 is a mechanism for starting the supply of the second reagent 46 from the second reagent holding part 45 to the carrier 2. Also as the second reagent supply mechanism 118, an actuator such as a solenoid is used similarity to the first reagent supply mechanism 116. The second reagent supply mechanism 118 is disposed at a position facing the second pressed part 12 of the loaded cartridge 100. The second reagent supply mechanism 118 is a pressing mechanism that applies a pressing force to the second pressed part 12 from the outside by pressing the second pressed part 12 of the cartridge 100. In a case where a pressing force is applied to the second pressed part 12 by the second reagent supply mechanism 118, the second reagent 46 is supplied from the second reagent holding part 45 to the carrier 2 by the above-described action. The second reagent supply mechanism 118 is used in both of the first assay flow and the second assay flow.

In addition to the loading part 112, the first reagent supply mechanism 116, and the second reagent supply mechanism 118, the assay apparatus 110 further includes a detection unit 114, a processor 120, and a memory 121 in the case body 111. In Fig. 6, the processor 120 and the memory 121 are illustrated outside the case body 111 of the assay apparatus 110, but this is a schematic diagram and the processor 120 and the memory 121 are actually disposed inside the case body 111.

The detection unit 114 optically detects the color development state of the assay region L1, the control region L2, and the color development region L3, and outputs a detection signal indicating the color development state to the processor 120. The detection unit 114 is image sensor such as a complementary metal oxide semiconductor (CMOS) image sensor and a charge coupled device (CCD) image sensor and images an observation region including an assay region L1, a control region L2, and a color development region L3. Then, the imaged image is output from the detection unit 114 to the processor 120.

In addition, as an example, a light source 115 such as a light emitting diode that illuminates the assay region L1, the control region L2, and the color development region L3 during the imaging is provided on both sides of the detection unit 114.

The processor 120 integrally controls each part of the assay apparatus 110. An example of the processor 120 is a Central Processing Unit (CPU) that performs various types of control by executing a program. The CPU functions as a control unit having a detection unit control unit 122, a color development state discrimination unit 123, a first reagent supply mechanism control unit 124, a second reagent supply mechanism control unit 125, a display control unit 126, and a timer 128 by executing a program. The memory 121 is an example of a memory connected to or built in the CPU as the processor 120. For example, a control program is stored in the memory 121. The processor 120 is realized by the CPU executing a control program.

In addition to the control program, the memory 121 stores setting information that is preset in order for the processor 120 to perform various types of control. As the setting information, information necessary for the color development state discrimination unit 123 to discriminate a change in the color development state is recorded. Examples of the setting information include a first setting time t1 that is preset, a second setting time t2 that is preset, and a number of times K that is preset in the assay flow which will be described later.

The detection unit control unit 122 controls the imaging timing by the detection unit 114.

The first reagent supply mechanism control unit 124 operates the first reagent supply mechanism 116 to control the first pressed part 11 to be pressed.

The second reagent supply mechanism control unit 125 operates the second reagent supply mechanism 118 based on the change in the color development state of the color development region to control the second pressed part 12 to be pressed.

The color development state discrimination unit 123 executes the color development region discrimination processing, the control region discrimination processing, and the assay region discrimination processing based on the detection signal output by the detection unit 114. As described above, the detection unit 114 outputs the captured image of the observation region including the assay region L1, the control region L2, and the color development region L3. The color development state discrimination unit 123 executes each of the above-described discrimination processing based on the captured image.

The color development region discrimination processing is a processing of discriminating, based on the image of the observation region, a change in the color development state of the color development region L3, as an example, whether or not the color has changed from a dark green color, which is the color before the reaction with the first reagent 41, to an orange color. "Presence" of the change in the color development state means that the first reagent 41 is developed to the color development region L3.

The "change in color development state" includes any of an aspect in which a first color different from the color of the carrier changes to another second color (that is, a color change), an aspect in which the color of the carrier changes to another color by developing a color different from that of the carrier (that is, color development), and an aspect in which the density of the color changes (that is, a change in density).

The processor 120 operates the second reagent supply mechanism 118 via the second reagent supply mechanism control unit 125 in a case where the color development state discrimination unit 123 discriminates that the color development state in the color development region L3 has changed.

The control region discrimination processing is a processing of discriminating presence or absence of a change in the color development state of the control region L2 based on the detection signal output by the detection unit 114. In the present example, since a line is expressed in the control region L2 by the labeling substance 53 being captured in the control region, or the silver amplification after capturing the labeling substance 53, presence or absence of the expression of the line in the control region L2 is discriminated. In a case where it is discriminated that the color development state of the control region L2 is changed, that is, the expression in the control region L2 is present, the color development state discrimination unit 123 executes the assay region discrimination processing of the next step.

The assay region discrimination processing is a processing of discriminating presence or absence of a change in the color development state of the assay region L1 based on the detection signal output by the detection unit 114. In the present example, since a line is expressed in the assay region L1 by the labeling substance 53 being captured in the assay region L1, or the silver amplification after capturing the labeling substance 53, presence or absence of the expression of the line in the assay region L1 is determined.

In a case where the color development state discrimination unit 123 discriminates that the change in the color development state in the assay region L1 is present, the processor 120 displays the assay result as "positive" on the monitor 119 via the display control unit 126. In addition, in a case where it is discriminated that the change in the color development state in the assay region L1 is absent, the assay result as "negative" is displayed on the monitor 119 via the display control unit 126.

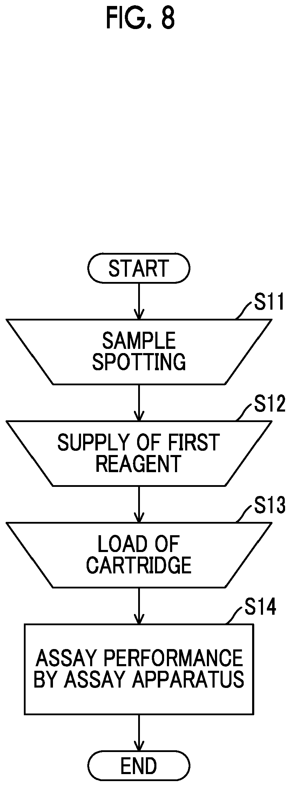

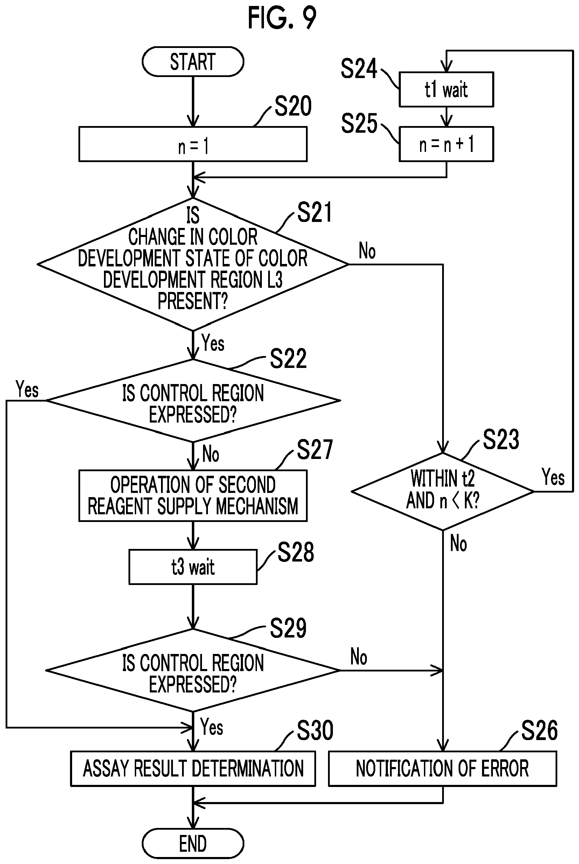

The procedure of the immunochromatographic assay using the assay apparatus 110 of the present embodiment will be described with reference to Fig. 8 and Fig. 9. Here, a first assay flow that is an aspect in which the supply of the first reagent 41 is performed by the user and the supply of the second reagent 46 is performed by the assay apparatus 110 will be described.

### (First assay flow)

Fig. 8 is a diagram showing the first assay flow.

First, a user adds dropwise the sample 50 from the dropping port 16 of the cartridge 100 onto the spotting region of the carrier 2 (Step S11).

Next, the user presses the first pressed part 11 of the cartridge 100 to start the supply of the first reagent 41 (Step S12).

After that, the user loads the cartridge 100 into the loading part 112 of the assay apparatus 110 in a state where a power is turned on (Step S13).

An assay of the loaded cartridge 100 is performed in the assay apparatus 110 (Step S14).

The time from the start of the supply of the first reagent 41 until the first reagent 41 is sufficiently developed in the carrier 2 varies depending on each cartridge, but it takes generally about 5 minutes to 10 minutes. The time from the pressing the first pressed part 11 by the user until the first pressed part 11 is loaded into the loading part 112 may be set according to the convenience of the user.

Fig. 9 shows a detailed assay flow of an assay performance (Step S14) by the assay apparatus 110 shown in Fig. 8.

By loading the cartridge 100 into the assay apparatus 110, the assay in the assay apparatus 110 (Step S14 in Fig. 8) is started.

As shown in Fig. 9, in the assay apparatus 110, first, n = 1 is set in the processor 120 (Step S20). Here, n is a parameter of the number of times of executing the discrimination processing of the color development region L3. The processor 120 discriminates whether or not the color development state of the color development region L3 is changed (specifically, the change from a dark green color to an orange color) (Step S21). Specifically, in a state where the observation region is illuminated by turning on the light source 115, the processor 120 causes the detection unit 114 to perform imaging by operating the detection unit 114. Then, the processor 120 acquires the captured image from the detection unit 114, and discriminates the change in the color development state of the color development region L3 from the acquired captured image. In a case where it is discriminated that a change in the color development state of the color development region L3 is present, that is, in a case where the color of the color development region L3 has changed from a dark green color to an orange color, it means that the first reagent 41 has reached the color development region L3, and the assay region L1 and the control region L2 which are on the upstream side of the color development region L3.

In a case where the color development state of the color development region L3 has changed (Step S21: Yes), the processor 120 discriminates whether or not a line is expressed in the control region L2 (Step S22). In Step S22, the processor 120 discriminates the change in the color development state of the control region L2 from the captured image. The processor 120 discriminates, for example, whether or not the color optical density of the control region L2 reaches a density not less than a preset reference, and in a case where the density is not less than the reference, the processor 120 discriminates that the control region L2 is expressed. The case where it is discriminated that the control region L2 is expressed means that the sample 50 has reached the control region L2 and the assay region L1 on the upstream side thereof, and are subjected to silver amplification, that is, the second reagent 46 has already been supplied.

On the other hand, in a case where the color development state of the color development region L3 has not changed (Step S21: No), determination of that the it is within the second setting time t2 and that the number of times n of discriminating the change in the color development state of the color development region L3 is less than K times (n < K) is performed (Step S23).

Here, in a case where the process has exceeded the second setting time t2 or it is not n < K (Step S23: No), the processor 120 notifies the error (Step S26) and ends the assay flow. For example, the notification of the error is performed by displaying an error message on the monitor 119. In addition to displaying the error message on the monitor 119, as a method of notifying the error, the error message may be notified by voice.

On the other hand, in a case where the it is within the second setting time t2 and it is n < K (Step S23: Yes), the process waits until the first setting time t1 elapses (Step S24). In Step S24 of Fig. 9, it is shown as "11 wait". The first setting time t1 is, for example, about 30 seconds, and the second setting time t2 is preset to, for example, 20 minutes or the like. After that, n is incremented by 1 (indicated by n = n + 1 in Fig. 9) (Step S25), and the process returns to Step S21 of discriminating again whether or not the color development state of the color development region L3 has changed.

In the discrimination of whether or not the control region L2 is expressed (Step S22), in a case where the control region L2 is expressed (Step S22: Yes), since the amplification has already been performed, it is possible to determine the assay result as it is. Therefore, the processor 120 determines the assay result by discriminating whether or not the assay region L1 is expressed as it is without amplification (Step S30), and ends the assay flow.

In the assay result determination, for example, the processor 120 discriminates whether the color optical density of the line-shaped assay region L1 reaches a density not less than a preset reference, and in a case where the density is not less than the reference, the processor 120 discriminates that the assay region L1 is expressed. The case where it is discriminated that the assay region L1 is expressed means that the sample 50 is positive, and the case where it is discriminated that the assay region L1 is not expressed means that the sample 50 is negative. In this way, the processor 120 determines the assay result of whether the sample 50 is positive or negative depending on the presence or absence of expression of the assay region L1.

On the other hand, in the discrimination of whether or not the control region L2 is expressed (Step S22), in a case where it is discriminated that the control region L2 is not expressed (Step S22: No), it is necessary to amplify the color development.

Therefore, the processor 120 operates the second reagent supply mechanism 118 to start the supply of the second reagent 46 (Step S27). In this way, the processor 120 operates the second reagent supply mechanism 118 only in a case where it is discriminated that the change in the color development state of the color development region L3 is present and the change in the color development state of the control region L2 is absent. In the present embodiment, the processor 120 presses the second pressed part 12 of the cartridge 100 by the second reagent supply mechanism 118. In a case where the second pressed part 12 is pressed, the second pressed part 12 is deformed to sink toward the second reagent holding part 45. Due to this deformation, the sheet member 48 of the second reagent holding part 45 is pressed against the protrusion 34 to break, and the second reagent 46 is supplied onto the carrier 2. After that, until the preset third setting time t3 elapses, the process waits the development of the second reagent 46 (Step S28). The third setting time t3 is set to, for example, about 3 minutes.

After the lapse of the third setting time t3, the processor 120 determines again whether or not the control region L2 is expressed (Step S29).

In the discrimination of whether or not the control region L2 is expressed in Step S29, in a case where the control region L2 is expressed (Step S29: Yes), the processor 120 determines the assay result by discriminating whether or not the assay region L1 is expressed (Step S30), and ends the assay flow.

In a case where it is discriminated that expression in the assay region L1 is present, the processor 120 that has performed the assay result determination displays the assay result as "positive" on the monitor 119. In addition, in a case where it is discriminated that expression in the assay region L1 is absent, the processor 120 displays the assay result as "negative" on the monitor 119.

On the other hand, in the discrimination of whether or not the control region L2 is expressed in Step S29, in a case where the control region L2 is not expressed (Step S29: No), an error is notified (Step S26) and the assay flow ends. In a case where the control region L2 is not expressed after the development of the second reagent 46, there is a possibility that the sample 50 has not been spotted. The assay flow in the assay apparatus 110 is as described above.

As described above, the assay apparatus 110 of the present embodiment includes a detection unit 114 that detects the color development state of the color development region L3 of the carrier 2 and a second reagent supply mechanism 118 for starting the supply of the second reagent 46 from the second reagent holding part 45 to the carrier 2. Then, the processor 120 operates the second reagent supply mechanism 118 based on the change in the color development state of the color development region L3. That is, the assay apparatus 110 of the present disclosure includes a processor 120 that controls the timing of operating the second reagent supply mechanism 118 based on a change in the color development state of the color development region L3 due to the reaction with the first reagent 41. Therefore, after the first reagent 41 is supplied to the carrier 2, even in a case where the development time until the first reagent 41 reaches the color development region L3 varies depending on the individual difference of the cartridge 100, the supply of the second reagent 46 to the carrier 2 can be started depending on the development time of each cartridge.

In a case where the supply timing of the second reagent 46 is temporally managed by using a timer as in the prior art, it is necessary to secure the maximum development time of the first reagent 41 in consideration of the individual difference of the cartridge 100, but according to the technique of the present disclosure, it is not necessary to wait for the maximum development time. Therefore, the occupancy time of the assay apparatus 110 by the cartridge 100 can be shortened according to a individual difference of the cartridge 100 as compared with the prior art in which the supply timing of the second reagent 46 is temporally managed by using a timer.

In the present disclosure, an actuator such as a solenoid is exemplified as the second reagent supply mechanism 118, but a mechanism capable of starting the supply of the second reagent 46 from the second reagent holding part 45 according to the configuration of the second reagent holding part 45 in the cartridge 100 may be used. For example, in a case where the second reagent holding part 45 includes a shutter and the supply of the second reagent 46 is started by opening the shutter, the second reagent supply mechanism 118 may be a mechanism for opening the shutter.

In the above-described embodiment, the first reagent 41 is the first amplifying liquid and the second reagent 46 is the second amplifying liquid, but the first reagent 41 and the second reagent 46 are not limited to this combination. A combination in which the first reagent 41 is a developing solution and the second reagent 46 is a cleaning liquid, or a combination in which the first reagent 41 is a developing solution or a cleaning liquid and the second reagent 46 is an amplifying liquid may be used.

However, the second reagent 46 is preferably an amplifying liquid that amplifies color development. In a case where the second reagent 46 is an amplifying liquid that amplifies the color development of the assay region L1, the color development of the assay region L1 is amplified, and thus the determination accuracy can be improved.

In addition, as in the present embodiment, the first reagent 41 and the second reagent 46 are preferably the amplifying liquids that amplify the color development of the assay region L1. In a case where the first reagent 41 and the second reagent 46 is an amplifying liquid that amplifies the color development of the assay region L 1, the color development of the assay region L1 is amplified, and thus the determination accuracy can be improved.

As described above, in the first assay flow, in the case where it is discriminated that change in the color development state is absent, after the discrimination, the processor 120 discriminates the presence or absence of the change in the color development state again after a elapse of the preset first setting time t1. Accordingly, even in a case where the development time until the first reagent 41 reaches the color development region L3 varies due to individual differences of the cartridges 100, a reliable assay can be performed according to the development state of individual cartridge 100.

In addition, in the first assay flow, in a case where the color development state does not change even after a lapse of a preset second setting time t2 from a preset time point after the cartridge 100 is loaded, or even after repeating the preset number of times of the discrimination, the processor 120 notifies an error. Accordingly, in a case where the color development state does not change even after a elapse of the second setting time or even after repeating the preset number of times of the discrimination, an error is notified, therefore in a case of the defective development of the first reagent or in a case where the first reagent is not developed, it is possible to prevent the apparatus from being occupied indefinitely.

In addition, the preset time point at which the count of the second setting time t2 is started may be the time when the cartridge is loaded, or the time when the preset number of times of the discrimination is ended. In addition, the preset number of times K may be appropriately set to 2 or more. In the present embodiment, it is determined whether or not the it is within t2 and whether or not the number of times of discrimination is less than the preset number of times (n < K) in Step S23, but only one of them may be determined. That is, only whether or not the it is within t2 may be determined to discriminate the color development state again or to notify the error. In addition, only whether or not it is n < K may be determined to discriminate the color development state again or to notify the error.

In the present embodiment, the detection unit 114 detects all the color development states of the assay region L1, the control region L2, and the color development region L3, but the detection unit may be provided individually for each region. That is, in a case where the color development state of the color development region L3 is defined as the first color development state, the color development state of the control region L2 is defined as the second color development state, and the color development state of the assay region L1 is defined as the third color development state, the first detection unit that detects the first color development state, the second detection unit that detects the second color development state, and the third detection unit that detects the third color development state each may be provided. However, as in the present embodiment, the first detection unit, the second detection unit, and the third detection unit are used commonly by one detection unit 114, and the detection unit 114 can detect the color development states of the three regions, and therefore the configuration can be simplified as compared with the case where the detection units are individually provided, and the cost can be suppressed. In addition, since only one detection unit 114 is required, space can be saved. The change in the color development state with respect to the assay region L1 and the control region L2 may be configured to be visually confirmed by the user.

In the first assay flow, in a case where it is discriminated that the change in the first color development state of the color development region L3 is present and the change in the second color development state of the control region L2 is present, the processor 120 discriminates the presence or absence of a change in the third color development state of the assay region L1. Then, the processor 120 operates the second reagent supply mechanism 118 only in a case where it is discriminated that the change in the first color development state of the color development region L3 is present and the change in the second color development state of the control region L2 is absent. Accordingly, in a case where the change in the second color development state and the development of the second reagent 46 is unnecessary, the presence or absence of the change in the third color development state of the assay region L1 can be discriminated without developing the second reagent 46, and therefore the occupancy time of the device can be shortened.

In addition, as described above, after operating the second reagent supply mechanism 118, in a case where the change in the second color development state of the control region L2 even after a lapse of a preset third setting time t3, the processor 120 is configured to notify an error. Accordingly, in a case where the second color development state does not change even after a lapse of the third setting time t3, an error is notified, therefore in a case of the defective development of the second reagent 46, it is possible to prevent the apparatus from being occupied wastefully.

In the present embodiment, the cartridge 100 includes a first reagent holding part 40 that holds the first reagent 41, and in the assay apparatus 110, the cartridge 100 in a state in which the supply of the first reagent 41 from the first reagent holding part 40 to the carrier 2 has started can be loaded. Therefore, before loading the cartridge 100, it is possible to start the supply of the first reagent to the carrier, for example, by an operation on the cartridge 100 by a user, and therefore, the occupancy time of the assay apparatus 110 can be shortened as compared with a case where the first reagent 41 is supplied to the carrier 2 after the cartridge 100 is loaded.

In the first assay flow described above, since the supply of the first reagent 41 is performed by pressing the first pressed part 11 by the user, the assay apparatus 110 may not include the first reagent supply mechanism 116.

### (Second assay flow)

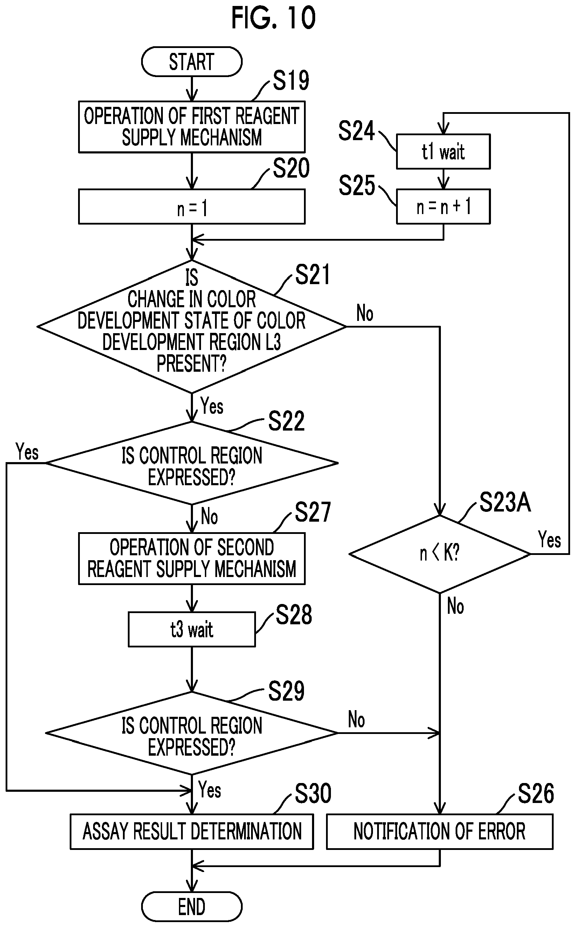

Fig. 10 shows a second assay flow. Only the points different from the first assay flow will be described in detail, and the same steps as those of the first assay flow is denoted by the same reference numeral for step, and detailed description thereof will be omitted.

In the first assay flow described above, the cartridge 100 is loaded into the loading part 112 of the assay apparatus 110 after the user performs the supply of the first reagent 41, but in the second assay flow, after the sample 50 is spotted by the user, the cartridge 100 is loaded into the loading part 112 without the supply of the first reagent 41.

By loading the cartridge 100 in a state where the first reagent 41 in the assay apparatus 110 is not supplied, the assay in the assay apparatus 110 is started.

The processor 120 first operates the first reagent supply mechanism 116 and starts the supply of the first reagent 41 to the carrier 2 (Step S19). The processor 120 lowers the first reagent supply mechanism 116 and presses the first pressed part 11 of the cartridge 100. The first pressed part 11 is deformed by pressing, and the protruding part 11b inside the first pressed part 11 pushes the liquid feeding pad 4 into the first reagent holding part 40. The sheet member 43 of the first reagent holding part 40 is pierced and the liquid feeding pad 4 is immersed in the first reagent 41, and the first reagent 41 is supplied to the carrier 2 from the liquid feeding pad 4.

Subsequent flow is substantially the same as that of the first assay flow. However, in the present configuration, in a case where there a change in the color development state of the color development region L3 (Step S21: No), in a case where the number of times n of discriminating the change in the color development state of the color development region L3 is less than K times (n < K) (Step S23A: Yes), the process waits the first setting time t1 (Step S24) and in a case where it is not n < K (Step S23A: No), an error is notified (Step S26). That is, unlike the case of the first assay flow, it is configured such that the process proceeds to the next step only depending on the number of times of discriminating the change in the color development state of the color development region L3 without determining whether or not the process has exceeded the second setting time t2.

In this way, since the assay apparatus 110 includes the first reagent supply mechanism 116 for starting the supply of the first reagent 41 from the first reagent holding part 40 to the carrier 2, as in the second assay flow, the supply of the first reagent 41 can also be performed in the assay apparatus 110, and operations other than the sample spotting can be automated. Also in the second assay flow in which the supply of the first reagent 41 is also performed by the assay apparatus 110, according to the present assay apparatus 110, the supply of the second reagent 46 to the carrier 2 can be started according to the development time of each cartridge. Accordingly, similarly to the case of the first assay flow, the occupancy time of the assay apparatus 110 by the cartridge 100 can be shortened according to a individual difference of the cartridge 100 as compared with the prior art in which the supply timing of the second reagent 46 is temporally managed by using a timer.

### <Modified example>

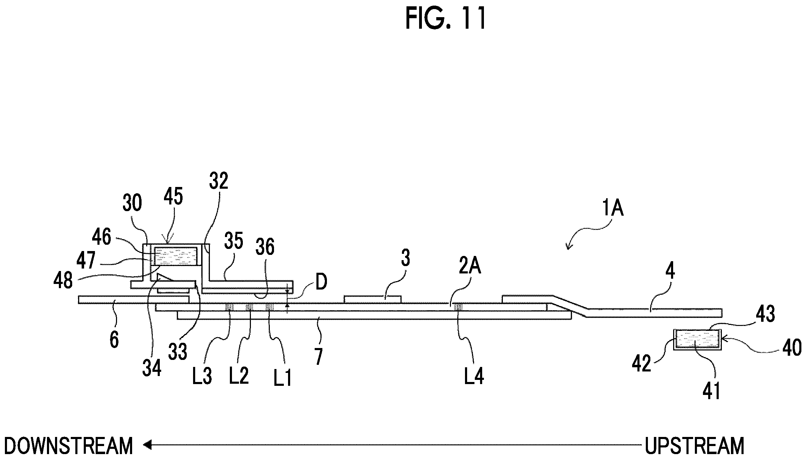

Fig. 11 is a side view of the assay strip 1A of the modified example. The same constituent elements as those of the above-described assay strip 1 are denoted by the same reference numerals, and detailed description thereof will be omitted.

The carrier 2A of the assay strip 1A includes an upstream-side color development region L4 disposed on the upstream side of the assay region L1 and between the first reagent holding part 40 and the assay region L1. In the present example, the color development region L3 disposed on the downstream side of the assay region L1 is referred to as a downstream-side color development region L3. Similarly to the downstream-side color development region L3, the color development state of the upstream-side color development region L4 changes by the reaction with the first reagent 41. Therefore, since the first reagent 41 reaches the upstream-side color development region L4 at an earlier stage as compared with the time from the start of the supply of the first reagent 41 to the development of the first reagent 41 to the downstream-side color development region L3, whether or not the supply of the first reagent 41 is started can be confirmed at an early stage.

In the housing encompassing the assay strip 1A, a portion facing the upstream-side color development region L4 is required to be formed of a transparent member that allows the upstream-side color development region L4 to be visually recognized.

In addition, in a case of using the cartridge including the assay strip 1A, the assay apparatus 110 includes, in addition to the detection unit 114 that detects the color development state of the downstream-side color development region L3, preferably the upstream-side detection unit that detects the color development state of the upstream-side color development region L4. As the upstream-side detection unit, for example, an image sensor can be used similarly to the downstream-side detection unit 114. In addition, in this case, the processor 120 is preferably configured to operate the first reagent supply mechanism 116 in a case where it is discriminated that a change in the color development state of the upstream-side color development region L4 is absent.

Here, a third assay flow will be described in which the cartridge provided with the assay strip 1A is used and the assay apparatus 110 including the upstream-side detection unit is used.

### (Third assay flow)

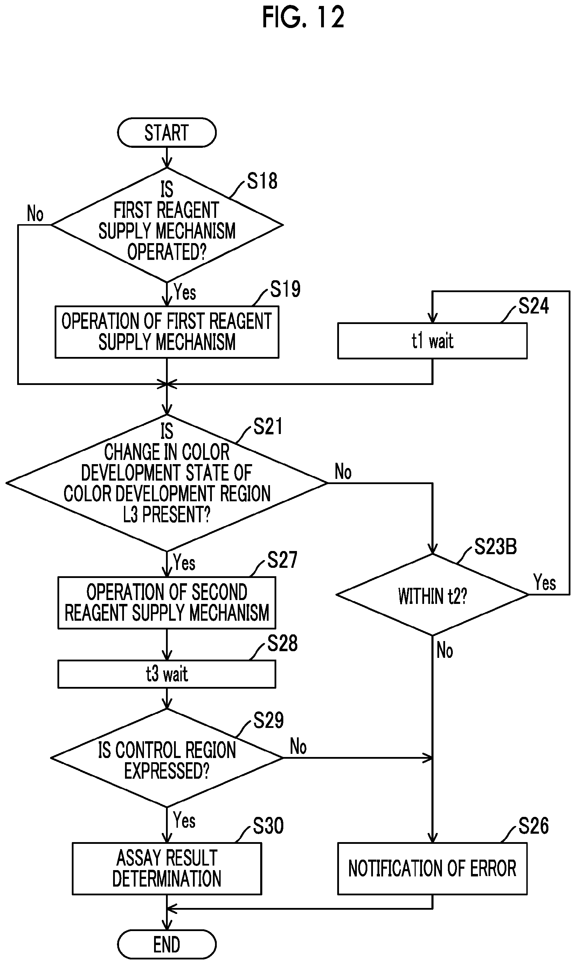

Fig. 12 shows a third assay flow. Only the points different from the first assay flow will be described in detail, and the same steps as those of the first assay flow is denoted by the same reference numeral for step, and detailed description thereof will be omitted.

In the third assay flow, first, it is discriminated whether or not the first reagent supply mechanism 116 is operated (Step S18). This is a step of discriminating whether or not the first reagent 41 is appropriately supplied. The processor 120 causes the upstream-side detection unit to detect the color development state of the upstream-side color development region L4, and discriminates whether or not a change in the color development state based on the color development state detected by the upstream-side detection unit. In a case where it is discriminated that a change in the color development state is absent, the processor 120 discriminates that the operation of the first reagent supply mechanism 116 is necessary (Step S18: Yes), and operates the first reagent supply mechanism 116 (Step S19). On the other hand, in a case where it is discriminated that the change in the color development state is present, the processor 120 discriminates that the operation of the first reagent supply mechanism 116 is unnecessary (Step S18: No).

After that, the processor 120 discriminates whether or not the color development state of the downstream-side color development region L3 has changed (Step S21). The subsequent flow is substantially the same as that of the first assay flow. However, in a case where the color development state of the downstream-side color development region L3 has not changed (Step S21: No), in a case where the it is within the second setting time t2 (Step S23B: Yes), the process waits the first setting time t1 (Step S24) and in a case where it had exceeded the second setting time t2 (Step S23B: No), an error is notified (Step S26). That is, unlike the case of the first assay flow, it is configured such that the process proceeds to the next step depending on whether or not it is within the second setting time t2 without counting the number of times of discriminating the change in the color development state of the color development region L3.

In addition, in the present third assay flow, in a case where it is discriminated that a change in the color development state of the downstream-side color development region L3 (Step S21: Yes), the second reagent supply mechanism 118 is operated (Step S27) without confirming the expression of the control region. In this way, in a case where it is discriminated that the change in the color development state of the color development region L3, the second reagent supply mechanism 118 may be operated to supply the second reagent 46 to the carrier 2 without confirming presence or absence of the expression of the control region.

Similarly to the above-described assay apparatus 110, the assay apparatus of the present design change example includes the processor 120 configured to control the timing at which the second reagent supply mechanism 118 is operated based on the change in the color development state of the color development region L3 due to the reaction with the first reagent 41. Therefore, the same effect as the assay apparatus 110 can be obtained. That is, the occupancy time of the assay apparatus 110 by the cartridge 100 can be shortened according to a individual difference of the cartridge 100 as compared with the prior art in which the supply timing of the second reagent is temporally managed by using a timer.

The carrier 2A provided in the cartridge of the design change example includes an upstream-side color development region L4 disposed on the upstream side of the assay region L1 and between the first reagent holding part 40 and the assay region L1. The color development state of the upstream-side color development region L4 changes by the reaction with the first reagent 41. In addition, the assay apparatus 110 of the design change example includes an upstream-side detection unit that detects a color development state of the upstream-side color development region L4. Then, the processor 120 operates the first reagent supply mechanism 116 in a case where it is discriminated that the change in the color development state of the upstream-side color development region L4. Therefore, the supply of the first reagent 41 is reliably performed in a case where the user has forgotten the operation of starting the supply of the first reagent 41 or the processing of supplying the first reagent is not appropriate, such as inadequate operation.

In the above-described design change example, whether or not the first reagent supply mechanism 116 is operated is determined by discriminating the change in the color development state of the upstream-side color development region L4. However, the determination of whether or not the first reagent supply mechanism 116 is operated is not limited to the present embodiment. In the assay apparatus 110, the processor 120 is configured to determine whether or not the operation of the first reagent supply mechanism is necessary depending on whether or not the first pressed part 11 of the cartridge 100 is in a pressed state. Specifically, the assay apparatus 110 may include a detection unit that detects whether or not the first pressed part 11 of the cartridge 100 is in a pressed state. Examples of the detection unit include an image sensor that images the first pressed part 11. Then, in a case where the processor 120 of the assay apparatus 110 discriminates that the first pressed part 11 is in the pressed state based on the image of the first pressed part 11 imaged by the image sensor, it may be determined that the operation of the first reagent supply mechanism 116 is unnecessary and in a case where the processor 120 discriminates that the first pressed part 11 is not in the pressed state, it may be determined that the operation of the first reagent supply mechanism 116 is necessary.

In the above-described embodiment, as the processor 120 and a hardware structure of a processing unit as internal configurations thereof that executes various types of processing, such as a detection unit control unit 122, a color development state discrimination unit 123, a first reagent supply mechanism control unit 124, and a second reagent supply mechanism control unit 125, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be formed of one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors.

Based on the above description, the following techniques can be grasped.

### [Appendix 1]

A method of operating an immunochromatographic assay apparatus including a loading part in which a cartridge including a carrier and a second reagent holding part is attachably and detachably loaded, the carrier having at least three regions of a spotting region on which a sample is spotted, an assay region in which a color development state changes depending on whether the sample is positive or negative, and a color development region in which a color development state changes by reaction with a first reagent, and the second reagent holding part holding a second reagent to be developed in the assay region after the first reagent has been developed in the color development region, a detection unit that detects a color development state in the color development region, a second reagent supply mechanism for starting a supply of the second reagent from the second reagent holding part to the carrier, and a processor, the method of operating an immunochromatographic assay apparatus comprising,
via the processor:
discriminating presence or absence of a change in the color development state of the color development region based on the change in the color development state of the color development region detected by the detection unit; and
operating the second reagent supply mechanism.

The disclosure of Japanese patent application 2021-050776 filed on March 24, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An immunochromatographic assay apparatus comprising:
a loading part in which a cartridge including a carrier and a second reagent holding part is attachably and detachably loaded, the carrier having at least three regions of a spotting region on which a sample is spotted, an assay region in which a color development state changes depending on whether the sample is positive or negative, and a color development region in which a color development state changes by reaction with a first reagent, and the second reagent holding part holding a second reagent to be developed in the assay region after the first reagent has been developed in the color development region;
a detection unit that detects a color development state in the color development region;
a second reagent supply mechanism for starting a supply of the second reagent from the second reagent holding part to the carrier; and
a processor configured to discriminate, based on a change in the color development state of the color development region, presence or absence of the change in the color development state of the color development region and operate the second reagent supply mechanism.

2. The immunochromatographic assay apparatus according to claim 1,
wherein at least the second reagent among the first reagent and the second reagent is an amplifying liquid that amplifies color development in the assay region.

3. The immunochromatographic assay apparatus according to claim 2,
wherein the first reagent and the second reagent are amplifying liquids that amplify color development in the assay region by reacting with each other.

4. The immunochromatographic assay apparatus according to any one of claims 1 to 3,
wherein in a case where the change in the color development state of the color development region is discriminated to be absent, after the discrimination, the processor is configured to discriminate the presence or absence of the change in the color development state of the color development region after a lapse of a preset first setting time.

5. The immunochromatographic assay apparatus according to any one of claims 1 to 4,
wherein in a case where the color development state of the color development region is not changed even after a lapse of a preset second setting time from a preset time point after the cartridge is loaded or even after repeating a preset number of times of discrimination, the processor is configured to notify an error.

6. The immunochromatographic assay apparatus according to any one of claims 1 to 5,
wherein in a case where a direction toward the assay region with respect to the spotting region is defined as a downstream side of the carrier, the carrier of the cartridge has a control region that is provided on a downstream side of the assay region and that shows a development of the sample supplied from the spotting region to the carrier in the assay region by a change in color development state,
in a case where the detection unit is defined as a first detection unit, the color development state detected by the first detection unit is defined as a first color development state, the color development state of the control region is defined as a second color development state, and the color development state of the assay region is defined as a third color development state,
the immunochromatographic assay apparatus includes a second detection unit that detects the second color development state of the control region, and
a third detection unit that detects the third color development state of the assay region, and
in a case where a change in the first color development state of the color development region is present and a change in the second color development state of the control region is discriminated to be present, the processor is configured to discriminate presence or absence of a change in the third color development state of the assay region.

7. The immunochromatographic assay apparatus according to claim 6,
wherein the processor is configured to operate the second reagent supply mechanism only in a case where the change in the first color development state of the color development region is present and the change in the second color development state of the control region is discriminated to be absent.

8. The immunochromatographic assay apparatus according to claim 6 or 7,
wherein after operating the second reagent supply mechanism, in a case where the change in the second color development state of the control region is absent even after a lapse of a preset third setting time, the processor is configured to notify an error.

9. The immunochromatographic assay apparatus according to any one of claims 1 to 8,
wherein the cartridge includes a first reagent holding part that holds the first reagent.

10. The immunochromatographic assay apparatus according to claim 9,
wherein the cartridge in a state where a supply of the first reagent from the first reagent holding part to the carrier is started is capable of being loaded.

11. The immunochromatographic assay apparatus according to claim 9 or 10, comprising:
a first reagent supply mechanism for causing the cartridge in a state of being loaded in the loading part to start a supply of the first reagent from the first reagent holding part to the carrier.

12. The immunochromatographic assay apparatus according to claim 11,
wherein in the cartridge, in a case where a direction toward the assay region with respect to the spotting region is defined as a downstream side of the carrier, the color development region is disposed on a downstream side of the assay region and the first reagent holding part is provided on an upstream side of the spotting region.

13. The immunochromatographic assay apparatus according to claim 11 or 12,
wherein in a case where the color development region disposed on a downstream side of the assay region is defined as a downstream-side color development region, the carrier of the cartridge has an upstream-side color development region which is on an upstream side of the assay region and is disposed between the first reagent holding part and the assay region, and in which a color development state changes by a reaction with the first reagent,
in a case where the detection unit is defined as a downstream-side detection unit that detects a color development state of the downstream-side color development region, the immunochromatographic assay apparatus includes an upstream-side detection unit that detects a color development state of the upstream-side color development region, and
in a case where a change in the color development state of the upstream-side color development region is discriminated to be absent, the processor is configured to operate the first reagent supply mechanism.
